# EUROPEAN PATENT SPECIFICATION

(11) **EP 2 085 076 B1**
(45) Date of publication and mention of the grant of the patent: **23.02.2011**
(21) Application number: 07825384.6
(22) Date of filing: 16.10.2007
(51) Int. Cl.: A61K 9/16, A61K 31/433, A61K 31/5415, A61K 9/50, A61K 9/58, A61K 9/48, A61K 9/20, A61K 9/26

(54) **PHARMACEUTICAL COMPOSITION IN THE FORM OF COATED MICROSPHERES FOR THE MODIFIED RELEASE OF A MUSCLE RELAXANT AND AN NSAID**
PHARMAZEUTISCHE ZUSAMMENSETZUNG IN FORM VON BESCHICHTETEN MIKROKÜGELCHEN ZUR MODIFIZIERTEN FREISETZUNG EINES MUSKELRELAXANS UND EINES NSAR
COMPOSITION PHARMACEUTIQUE SOUS FORME DE MICROSPHÈRES ENROBÉES POUR LA LIBÉRATION MODIFIÉE D'UN MYORELAXANT ET D'UN AINS

(30) Priority: 18.10.2006 MX LL06012024
(43) Date of publication of application: 05.08.2009
(73) Proprietor: Laboratorios Senosiain, S.a. De C.V., Distrito Federal 11560 (MX)
(72) Inventor: GARCIA-SALGADO LOPEZ, Enrique Raúl, D.F., 11320 (MX); ARZOLA PANIAGUA, Angélica, D.F., 11320 (MX); POOT LÓPEZ, Luis Fernando, D.F., 11320 (MX); ESCORCIA RODRÍGUEZ, Francisco, D.F., 11320 (MX); BARRANCO HERNANDEZ, Gustavo, D.F., 11320 (MX)
(74) Representative: Carpintero Lopez, Francisco
(86) International application number: PCT/IB2007/003083
(87) International publication number: WO 2008/047208

(56) References cited:
- FR-A1- 2 735 369
- US-A1- 2004 204 413
- US-A1- 2005 100 594

## Description

### FIELD OF THE INVENTION

The invention relates to a modified release pharmaceutical composition in capsules with coated microspheres, which comprises the combination of two active ingredients with radically different plasma concentration times, namely a muscle relaxant (tizanidine), and a non-steroidal anti-inflammatory drug (meloxicam), and pharmaceutically acceptable excipients or vehicles; as well as a process for producing the composition and the use of said combination for the preparation of a drug having synergic therapeutic effect in the treatment of spasticity, disorders related to skeletal muscle and/or muscular ailments and moderate to severe pain in general.

### BACKGROUND OF THE INVENTION

Muscular disorders are a wide spread condition among general population causing stiffness, muscular contraction and pain, and muscular disorders also interfere with muscular movement and function (including but not limiting to walking, handling, balance, talking, swallowing).

Tizanidine is a central action 2-alpha adrenergic agonist, is well tolerated and is beneficial in the treatment of muscular spasticity of diverse etiology. In the spinal region, the tizanidine decreases the reflex activity, specially the polysynaptic reflex activity; tizanidine can repair or enhance the presynaptic noradrenergic inhibition in spastic patients and tizanidine further provides relief for spasms and muscular tone caused by affections such as multiple sclerosis or spinal injury. Furthermore, tizanidine has antispastic effects even on frequently used drugs, such as baclofen, diazepam or clonazepam, but tizanidine does not cause the resistance effects of these drugs. Furthermore, it has been shown that tizanidine may present other effects, such as decreasing rebound cephalalgy due to detoxification of analgesic drugs; seemingly, tizanidine is effective in the treatment of chronic headache and tizanidine seems to have fewer properties as hypertensive agent.

After oral administration, tizanidine is completely absorbed, it reaches its maximum plasma concentration (Cmax) at 1.5 hours after its administration and has a half-life time of approximately 2.5 hours. Due to the short half-life time of this drug, said drug must be administrated every 6 or 8 hours and shows linear pharmacokinetics in the range of 1 to 20 mg. The excretion of tizanidine, is mainly 60% in urine and about 20% in feces (PDR information, 2006). Depending on the condition, the dose may be 2 mg for 3 or 4 times a day or, usually, the dose may be greater than 24 mg divided in 3-4 administrations per day, and the maximum recommended dose is 36 mg per day.

Tizanidine may cause side effects such as: dizziness and weakness, as well as lightheadedness, stomach upset, vomit, tickling in arms, legs, hands and feet, feeling of dry mouth, stronger muscular spasms and severe muscular contraction.

Meloxicam is a non-steroidal anti-inflammatory drug (NSAID), is a selective inhibitor of COX-II, derived from enolic acid, and has anti-inflammatory action and a good tolerability profile. Meloxicam is indicated in the treatment of acute and chronic rheumatoid arthritis, osteoarthritis (degenerative articulation disease), shoulder and hip peri-arthritis and muscular swelling, as well as in the treatment of gout, swelling and pain, pain due to traumas, soft tissue inflammatory processes (airways), gynecological conditions and primary dysmenorrhea.

The absolute bioavailability of meloxicam is 89% and it has been shown that the pharmacokinetics, after intravenous administration, is linear in the range of 5 to 60 mg. The elimination half-life time of meloxicam is variable from 15 to 20 hours and it has been recorded to be consistent at different therapeutic doses of meloxicam, which is an indication of a linear metabolism in the therapeutic range of this drug (Gates et al., 2005; PDR Information, 2006). The maximum plasma concentration is reached at 4-5 hours after administration, which is an indication of slow absorption (Carrasco-Portugal et al., 2005). Additionally, a second concentration peak is seen at 12-14 hours after administration, which indicates a gastrointestinal re-circulation (PDR Information, 2006).

It has been recorded that the effective doses for therapeutic indications are 7.5 and 15 mg per day. It should be noted that a potentially larger effect of 22.5 mg of meloxicam has been evaluated. However, only an increase in gastrointestinal adverse effect was observed. Hence, the recommended daily dose for meloxicam is 15 mg (Ahmed et al., 2005).

The FDA and the British Health Committee state that, most of the non-steroidal anti-inflammatory drugs used today are responsible for causing gastric injuries. Meloxicam causes side effects and, such as other NSAIDs, these effects are mainly gastrointestinal symptoms such as: pyrosis (heart burn), diarrhea, throat ache, cough, rhinorrhea (runny nose), dyspepsia, nausea, vomit, constipation, gastrointestinal ulcer, macroscopic or microscopic gastrointestinal bleeding, transitory anomalies of hepatic function parameters, alteration of renal function parameters, pruritus, skin rash and photo sensibility.

For the combination of the invention, tizanidine and meloxicam, there is no competition for the metabolic paths. Tizanidine acts centrally, with its main place of action being the spinal cord, decreasing muscular tone, and having muscle-relaxing features, as well as having a moderate central analgesic effect. Meloxicam acts on the inhibition of prostaglandins, acting mainly on the oxygenase II coenzyme (COX-II), which is the main cause of pain; the little action of meloxicam on COX-I decreases gastric and renal disorders.

The formulation provides a combination with a synergic effect and the muscle-relaxing activity of a non-steroidal anti-inflammatory drug. Said formulation allows for the treatment of muscular spasticity, disorders related to skeletal muscle and/or muscular ailments and moderate to severe pain in a single dose. This synergy may allow to perform posology 1-2 times a day and/or to decrease the active ingredient concentration in the formulation with the benefit that this formulation synergy allows for the decrease of adverse effects. It must be noted that the new combination shows a synergic effect, which is translated into a higher muscle-relaxing activity, and a greater anti-inflammatory analgesic effect.

Some of the preferred embodiments of the composition refer to the following doses of tizanidine and meloxicam: 6 mg and 7.5 mg, 6 mg and 15 mg, 12 mg and 15 mg, respectively. Overall, the relation between tizanidine and meloxicam varies from 83%-16%, i.e., while tizanidine will vary between 0.5 to 36 mg per dose, meloxicam will vary from 2 mg to 15 mg.

International application PCT/US85/02335 refers to a pharmaceutical composition and a method to use said composition in the treatment of skeletal muscle disorders. Said composition comprises a muscle relaxant plus a non-steroidal anti-inflammatory drug. The skeletal muscle drug is selected from metocarbamol, carisoprodol or diazepam. The analgesic is selected from piroxicam, sudoxicam or isoxicam.

Unlike the composition revealed in the international application PCT/US85/02335, the composition of this invention is safer and more efficient, because in said international application carisoprodol is included as a muscle-relaxing drug. The use and abuse of carisoprodol has caused a few reported deaths, because carisoprodol may cause respiratory depression (Davis G. SMJ, USA, 2003). In the description of international application PCT/US85/02335 the pharmaceutical form of conventional tablets is mentioned. Said international application does not claim any particular pharmaceutical form nor does said international application mention specifically meloxicam as non-steroidal anti-inflammatory drug. Consequently it is noted that said application refers to the use of tablets, unlike this invention that refers to capsules with modified release coated microspheres and modified release tablets.

International application PCT/IB2004/001184 refers to a pharmaceutical formulation, preferably in modified release tablets, which formulation is constituted by a modified release muscle relaxant and a quick release or immediate release oxygenase 2 (COX-II) cycle inhibitor, preferably valdecoxib. Said invention is characterized in that the muscle relaxant is formulated and elaborated separately, i.e., composition 1 includes the COXII inhibitor and composition 2 includes the muscle relaxant; later, composition 2 is added to composition 1. Said international application describes formulations which include valdecoxib, celicoxib, paracoxib, etoricoxib, or a mixture thereof, as COX-II inhibitor. It should be noted that all the examples included in the application PCT/IB2004/001184 refer to valdecoxib. It is important to consider that, throughout the text of said international application, no mention is made of the compound meloxicam.

It should be noted that pharmacokinetic interactions between tizanidine and rofecoxib have been described, as rofecoxib inhibits the metabolism of tizanidine, causing an accumulation thereof and the occurrence of adverse events. This interaction has limited the use of the combination of tizanidine with rofecoxib. The aforementioned does not happen in this invention because no pharmacokinetic interaction between tizanidine and meloxicam exists.

Unlike international application PCT/IB2004/001184, this invention refers specifically to modified release capsules with microspheres. This invention comprises a modified release, particularly called "repeated release", of muscle relaxant, specifically tizanidine, and an immediate release non-steroidal anti-inflammatory drug, specifically meloxicam. The form in which this formulation is designed allows for the immediate release, in a first stage, of meloxicam and a portion of tizanidine from 40 to 60%; then, in no less than 2 hours, in a second stage, the remaining tizanidine is released. With this mechanism, the therapeutic scope is considerably improved and the potential adverse effects are decreased, because the plasma concentration of tizanidine is maintained and important concentration variations are avoided. In this invention, a continued process was developed, which process does not include independent pre-formulations, resulting in decreased manufacturing times and costs.

The design of the abovementioned composition provides a composition that maintains therapeutic activity during at least 12 hours in a row, because the plasma concentrations required for the active ingredients are maintained to achieve an optimal therapeutic effect.

International application PCT/CR02/000001 refers to a pharmaceutical formulation, preferably in tablets, which contains a non-steroidal anti-inflammatory drug that selectively inhibits COX-II, and a muscle relaxant to treat pain, especially muscular pain. Although several compounds are mentioned, as COX-II inhibitors and muscle relaxants, all the examples refer to the combination of rofecoxib with pridinol, unlike the composition of this invention, which makes reference to a specific combination of tizanidine and meloxicam and the manufacturing process to obtain modified release capsules with microspheres, where the tizanidine has a modified release and meloxicam has a quick or immediate release, which combination is useful in the treatment of muscular spasticity, disorders related to skeletal muscle and/or muscular ailments and moderate to severe pain in general.

Unlike this invention, presented in the form of modified release capsules with microspheres, currently, compositions containing a NSAID-type drug and a muscle relaxant in the form of immediate release tablets are available in the market. These products include mixtures of carisoprodol with meloxicam and metocarbamol with meloxicam; said compositions show considerable gastric interactions and swallowing problems due to the amount of active ingredient included in said products.

Furthermore, it is important to consider that, when administrating a drug in the form of microspheres, the potential gastric injuries are decreased, because the microspheres are distributed in a broader contact surface in the gastric tract. In the case of conventional tablets, they are deposited in a more restricted section of the gastric tract, which may cause an injury thereto.

For the reasons described above, there is a need to find a pharmaceutical composition containing a muscle relaxant (tizanidine) and a non-steroidal anti-inflammatory drug (meloxicam), and which may be administered one or two times a day.

Surprisingly, by this invention, a modified release composition has been devised wherein two active ingredients coexist showing a marked difference in plasma concentration times (Tmax) and half-life times, and which composition may be administered once or twice a day.

The composition of this invention is useful in the treatment of spasticity, disorders related to skeletal muscle and/or muscular ailments and moderate to severe pain in general.

By reason of the different plasma concentration times and the different half-life time of meloxicam and tizanidine, there is a need to delay the release of tizanidine to obtain similar plasma concentrations of said active ingredients and to achieve a continued therapeutic activity of the combination.

It is important to note that this invention provides a process that allows having a composition wherein the release of tizanidine is modified without affecting the release of meloxicam.

For this invention, a modified release process was developed, which process prevents the occurrence of plasma concentration peaks and maintains constant levels of therapeutic plasma concentrations, which decreases the incidence of adverse effects.

It has been recorded that, for the treatment of spasticity, substances such as baclofen and diazepam are used, and they cause tolerance and sedation on the patient, which disadvantages are not present with the combination of this invention.

### BRIEF DESCRIPTION OF THE DRAWINGS

The attached figures illustrate the behavior of the compositions of this invention when said compositions are administered.
Fig. 1 is the expected immediate or quick release profile.
Fig. 2 shows the modified release profile for the composition of this invention.
Fig. 3 shows the design of the composition.

### DESCRIPTION OF THE INVENTION

This invention provides a pharmaceutical composition that is useful in the therapeutic treatment of disorders related to spasticity, disorders related to skeletal muscle and/or muscular ailments and moderate to severe pain in general.

The proposed composition is an orally administered formulation that makes reference to a composition constituted by a muscle relaxant plus a non-steroidal anti-inflammatory drug in the form of modified release capsules with coated microspheres. The drugs may be released in two ways: (1) quick or immediate release, and (2) modified release, which in turn is sub-divided into sustained release, programmed release, repeated release, etc.

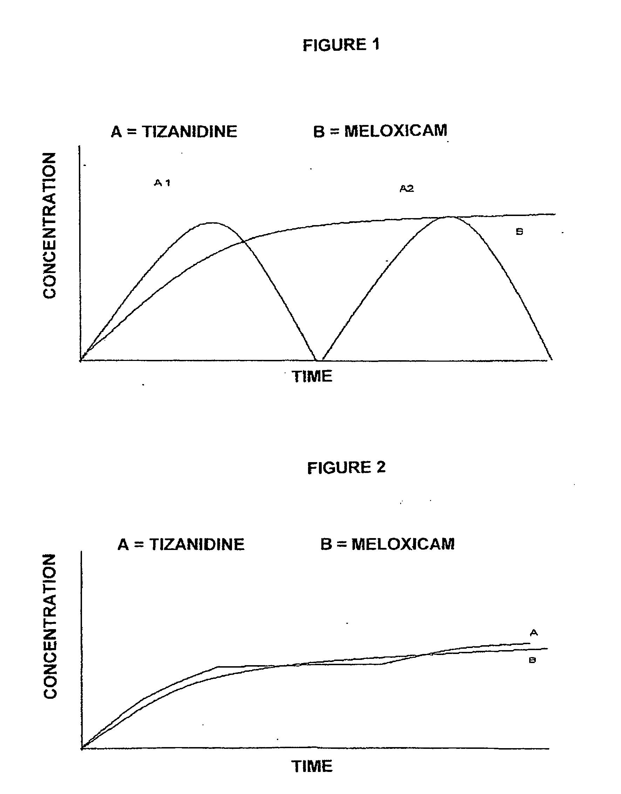

Figure 1 describes an absorption curve that shows the modified release pharmaceutical forms. In this graph, the behavior of two drugs is represented, the drugs reach their maximum plasma concentration time at considerably different times. "A" represents the behavior of a drug that reaches the maximum plasma concentration (Cmax) in a short time, whereas "8" reaches Cmax in a longer time. Hence, to maintain the therapeutic effect of "A" during the period required by "B", a double administration of "A" is needed, which in the graph is shown as "A1" and "A2".

The modified release pharmaceutical forms are those designed in such a way that either the rate at which or the place where the active ingredient is release is modified, in comparison with the immediate release pharmaceutical forms for the same active ingredient. One of the types of modified release is repeated release, wherein the extended release dosing forms release fractions of the active ingredient at certain intervals of time.

In this invention, a composition was formulated to contain tizanidine and meloxicam allowing to keep the plasma concentration requirements so as to obtain the desired therapeutic effect as shown in figure 2. This graph shows the behavior of two drugs that reach their corresponding maximum plasma concentration times at considerably different times, and which drugs have been administered jointly, and which are formulated in a repeated release pharmaceutical form, wherein: "A" represents the behavior of a drug that reaches Cmax in a short time, and "B" represents the behavior of a drug which reaches Cmax in a considerably longer period of time. Drug "A" has two release periods. The graph shows how "A" reaches a plasma concentration level and, later, a second therapeutic dose of "A" is released; hence, the release time of "A" is similar to the behavior of drug "B".

The process of this invention is characterized by shorter operational times compared to a similar process for coating the active ingredients or to other process which require a pre-treatment of the active ingredients in order to be included in one same formulation within one same enteric coating.

The process of this invention allows for a controlled release of the active ingredient tizanidine without affecting the immediate release of meloxicam.

It should be noted that handling low concentrations of active ingredient represents a technical problem to obtain microspheres that comply with content uniformity and formulation dose uniformity requirements; however, in the process of this invention, a methodology is developed to allow the control of tizanidine release, which is considered to be in low concentration in the composition.

In this invention, tizanidine is presented in two different layers divided by a delaying polymeric film; each of said films having a [sic] of about 3 mg of active ingredient. However, this invention conveniently complies with the content uniformity and dose uniformity requirement. The process of this invention decreases the release of powders, which results in high efficiency of the formulation.

Another advantage of the process of this invention is that work is performed at temperatures near room temperature, which results in energy savings and prevents potential degradation of the active ingredient and excipients in this innovative composition.

The composition is a repeated release composition, and this constitutes the preferred embodiment of the invention. Said preferred embodiment is characterized by a microsphere coated with polymeric layers (semi-permeable membrane) over an inert core; the diffusion of the drug depends on: type of membrane, thickness, pH, and the site where the drug is to be released. Said layers are constituted by an adhesive polymer, preferably Hydroxy propyl methyl cellulose (HPMC) plus the drug, followed by a film which is not soluble in acid pH or a semi-permeable film, and finally, a second layer of the adhesive polymer. This invention refers to a pharmaceutical composition in capsules, which composition is characterized by the coated microspheres comprising:
a) inert cores coated with a first film formed by a muscle relaxant, at least one adhesive polymer and at least one plasticizer agent;
b) a second delaying polymeric film, at least one plasticizer agent and a regulating solution; and
c) a third film formed by a NSAID drug, the muscle relaxant of the first film, at least one adhesive polymer, at least one plasticizer agent and at least one surfactant, wherein the muscle relaxant is a modified release muscle relaxant and the NSAID drug is an immediate release drug.

The first film contains the delayed-release drug portion and the third film is comprised of the two active ingredients to be released in a quick or immediate fashion.

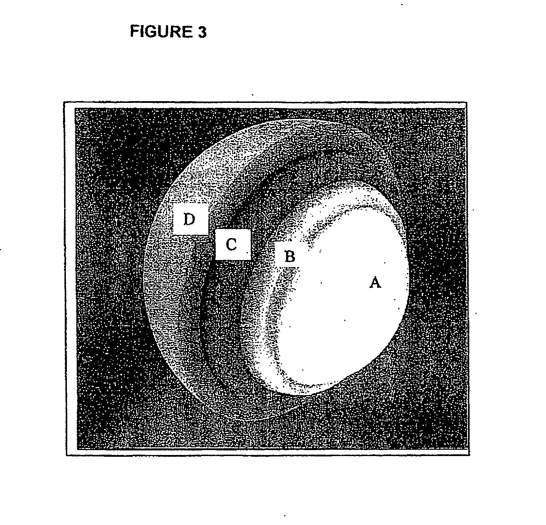

Figure 3 shows the design of the composition that shows the microsphere of this invention, wherein: "A" is the inert core; "B" is the first film formed by tizanidine or any pharmaceutically acceptable salts thereof (from 40% to 60% of the total content of tizanidine in this film), at least one adhesive polymer and at least one plasticizer agent; "C" is the second delaying film, at least one plasticizer agent and buffer solution; and "D" is the third film formed by meloxicam, tizanidine (from 40% to 60% of the total content of tizanidine in this film) or any pharmaceutically acceptable salts thereof, at least one adhesive polymer, at least one plasticizer agent, at least one surfactant agent and other excipients.

In this invention a drug is formulated which is presented in the form of microspheres (inert cores) with active ingredients at relatively low doses, particularly tizanidine, which is the active ingredient at the lowest concentration and which is divided into two layers or films. Working with relatively low active ingredient concentrations (6 mg) implies a number of difficulties, especially when the target is a controlled release, because it is essential to confirm that said active ingredient is evenly distributed. To achieve the aforementioned, it is important to determine the operational and process conditions that may allow us to effectively adhere said active ingredient. On the other hand, the in vitro quantification of the active ingredient release requires the development of an analytical methodology sensible enough to quantify low concentrations.

### FORMULATIONS

The formulation of modified release tablets and capsules with microspheres, as well as the manufacture process of the pharmaceutical combination of tizanidine and meloxicam will be described below.

The composition is characterized by the combination of tizanidine (or any pharmaceutically acceptable salts thereof), and meloxicam (or any pharmaceutically acceptable salts thereof), and any pharmaceutically acceptable vehicles or excipients.

The following are the preferred excipients or vehicles for this invention:
- Inert core base formed by cellulose or sugars selected from: lactose, glucose, dextrose or sucrose. This base shall provide support to the active ingredients and to the vehicles or excipients of the microsphere.
- Adhesive or binding polymer, selected from: hydroxy propyl cellulose, pre-gelatinized starch or hydroxy propyl methylcellulose. This polymer provides the microsphere with body and cohesion.
- Plasticizer agent, selected from propylene glycol or polyethylene glycol 20000. Said plasticizer agent provides the microsphere with strength and plasticity.
- Delaying polymer, selected from methacrylate derivates such as: Eudragit S 100, Eudragit RS, Eudragit RL, Eudragit L30D55, Eudragit 100 55 or Eudragit L 100. This polymer is an enteric material which covers and protects the microsphere in order to: enhance resistance to handling, mask any unpleasant flavor or smell and to improve appearance and stability during storage.
- Anionic or cationic surfactant agent to help spread and lubricate both the active substances and the delaying polymer and to allow for an easy application during the coating process.
- Acid or alkaline buffer solution selected from: hydrochloric acid, acetic acid, sodium hydroxide or ammonium hydroxide solutions.
- Other excipients or vehicles which may be used are microcrystalline cellulose, lactose, sodium fumarate, colorant and flavoring agent.

The composition obtained with this invention contains a dose range of the active ingredients from 0.5% to 36% for tizanidine and from 2.0% to 15% of meloxicam, plus pharmaceutically acceptable excipients in ranges that may be modified and adapted depending on active ingredient concentration in the formulation.

### Example 1: General formulation of capsules with microspheres

| **Active ingredients and excipients** | **Percentage** |
|---|---|
| Active ingredient 1. Muscle relaxant | 0.5 - 36.0 |
| Active ingredient 2. AINE COXII inhibitor | 2.0 - 15.0 |
| Inert cores | 37.0 - 75.0 |
| Binding agent | 1.5 - 6.0 |
| Enteric material (methacrylates) | 7.0 - 11.1 |
| Plasticizer 1 | 1.0- 2.0 |
| Surfactant | 0.2 - 0.5 |
| Plasticizer 2 | 1.2 - 3.0 |
| Buffer | - - - |
| Water | - - - |
| Total | 100 |

### Example 2: Proposed formulation of capsules with microspheres

| **Active ingredients and excipients** | **Percentage** |
|---|---|
| Tizanidine (active ingredient 1) | 0.5 |
| Meloxicam (active ingredient 2) | 2.0 |
| Inert cores | 75.0 |
| Hydroxy propyl methyl cellulose | 6.0 |
| Eudragit S-100 | 11.1 |
| Triethyl citrate (Eudraflex-2) | 2.0 |
| Sodium lauryl sulphate | 0.5 |
| Polyethylene glycol | 3.0 |
| Water* | - - - - |
| Ammonium hydroxide* | - - - |
| Total | 100 |

### Example 3: Formulation of capsules with microspheres

| **Active ingredients and excipients** | **Percentage** |
|---|---|
| Tizanidine | 36.0 |
| Meloxicam | 15.0 |
| Inert cores | 38.0 |
| Hydroxy propyl methyl cellulose | 1.5 |
| Eudragit S-100 | 7.0 |
| Triethyl citrate (Eudraflex-2) | 1.0 |
| Sodium lauryl sulphate | 0.2 |
| Polyethylene glycol | 1.0 |
| Water* | - - - |
| Ammonium hydroxide* | - - - |
| Total | 100 |

### Example 4: Formulation of capsules with microspheres.

| **Active ingredients and excipients** | **Percentage** |
|---|---|
| Tizanidine | 6.0 |
| Meloxicam | 15.0 |
| Inert cores | 68.0 |
| Hydroxy propyl methyl cellulose | 1.5 |
| Eudragit S-100 | 7.0 |
| Triethyl citrate (Eudraflex-2) | 1.0 |
| Sodium lauryl sulphate | 0.2 |
| Polyethylene glycol | 1.0 |
| Water* | - - - |
| Ammonium hydroxide* | - - - |
| Total | 100 |

### Example 5: Formulation of capsules with microspheres

| **Active ingredients and excipients** | **Percentage** |
|---|---|
| Tizanidine | 12.0 |
| Meloxicam | 15.0 |
| Inert cores | 62.0 |
| Hydroxy propyl methyl cellulose | 1.5 |
| Eudragit S-100 | 7.0 |
| Triethyl citrate (Eudraflex-2) | 1.0 |
| Sodium lauryl sulphate | 0.2 |
| Polyethylene glycol | 1.0 |
| Water* | - - - |
| Ammonium hydroxide* | - - - |
| Total | 100 |

### Example 6: Formulation of tablets

| **Active ingredients and excipients** | **Percentage** |
|---|---|
| Tizanidine | 1.0 - 1.3 |
| Meloxicam | 1.3 - 1.6 |
| Inert cores | 12.5 - 15.4 |
| Hydroxy propyl methyl cellulose | 0.9 - 1.1 |
| Eudragit S-100 | 1.9 - 2.4 |
| Triethyl citrate (Eudraflex-2) | 0.2 - 0.4 |
| Sodium lauryl sulphate | 0.07 - 0.09 |
| Polyethylene glycol | 0.3 - 0.5 |
| Cellulose | 27.0 - 33.0 |
| Stearyl sodium fumarate | 1.8 - 2.2 |
| Lactose in a quantity sufficient for (q.s.) | 100% |

### Example 7: Preferred embodiment for the formulation of capsules with microspheres

| **Active ingredients and excipients** | **Percentage** | **mg per 100 mg of composition** |
|---|---|---|
| Tizanidine (Active ingredient A) | 6.00 | 6.00 |
| Meloxicam (Active ingredient B) | 7.50 | 7.50 |
| Inert cores | 66.60 | 66.60 |
| HPMC | 4.90 | 4.90 |
| Eudragit S-100 | 10.70 | 10.70 |
| Triethyl citrate (Eudraflex-2) | 1.60 | 1.60 |
| Sodium lauryl sulphate | 0.37 | 0.37 |
| Polyethylene glycol | 2.15 | 2.15 |
| Water | - - | - - |
| Ammonium hydroxide | - - | - - |
| Total | 100 | 100 |

### Manufacture of the formulation of capsules with microspheres.

Make sure the materials and equipment match the manufacture of the formulation. The preparation process for the preferred formulation is as follows:
1. The components of the formula are weighted.
2. The selected adhesive or binding polymer is dissolved.
3. The appropriate part of the active ingredient to be released in two stages (active ingredient "A") is added to the mixture of step 2, and the mixture is mixed to homogeneity.
4. The plasticizer is added to the preparation of step 3, and the mixture is mixed to homogeneity.
5. Separately, the inert cores are placed on the fluidized bed equipment and agitation starts.
6. Spraying of preparation of step 4 is started.
7. Separately, a solution of the delaying polymer Eudragit S-100, plasticizer and, if needed, sodium hydroxide buffer to adjust pH is prepared, and mixed to homogeneity.
8. The preparation in step 7 is sprayed on the coated cores.
9. Separately, a solution containing binder, plasticizer and surfactant is prepared.
10. The rest of the active ingredient "A" and all the active ingredient "B" are added to the preparation of step 9.
11. Water is added into the mixture obtained in step 10, and the mixture is then agitated to homogeneity.
12. The preparation of step 11 is sprayed on the coated cores of step 8.
13. The product is dried to meet the required conditions.
14. The product is encapsulated.

The process to elaborate the coated microspheres of this invention is characterized by the coating steps being performed continuously and at room temperature. During said process, the following is sprayed on the inert cores:
a) a preparation of the muscle relaxant, at least one adhesive polymer, at least one plasticizer, and at least one surfactant.
b) a second film formed by a delaying polymer film, at least one plasticizer, and at least one buffer solution; and
c) a third film formed by a NSAID, the muscle relaxant, at least one adhesive polymer, at least one plasticizer, and at least one surfactant.

This invention provides a composition of a muscle relaxant by a non-steroidal anti-inflammatory drug for the treatment of spasticity, disorders related to skeletal muscle and/or muscular ailments and moderate to severe pain in general.

Said drug may be administered once or twice a day, and also offers a better control of plasma drug levels, wherein the incidence and severity of side effects of both drugs are reduced, and the gastric irritation caused by the conventional release compacted drugs is decreased by reason of the characteristics of the modified release and the use of the protective gastric features of tizanidine.

## Claims

1. A pharmaceutical composition **characterized by** the coated microspheres comprising:
a) inert cores coated with a first film formed by a muscle relaxant, at least one adhesive polymer, and at least one plasticizer;
b) a second delaying polymer film, at least one plasticizer, and a regulating solution; and
c) a third film formed by a NSAID, the muscle relaxant of the first film, at least one adhesive polymer, at least one plasticizer, and at least one surfactant;
wherein the muscle relaxant shows a modified release, and the NSAID shows immediate release.

2. The pharmaceutical composition in accordance with claim 1, wherein the muscle relaxant shows plasma concentration times and half-life time which are different from the plasma concentration time and half-life time of the NSAID.

3. The pharmaceutical composition in accordance with claim 1, wherein the muscle relaxant is tizanidine or any pharmaceutically acceptable salts thereof.

4. The pharmaceutical composition in accordance with claim 1, wherein the NSAID is meloxicam or any pharmaceutically acceptable salts thereof.

5. The pharmaceutical composition in accordance with claim 3, wherein the concentration of tizanidine or any pharmaceutically acceptable salts thereof is 0.5%-36% per dose unit.

6. The pharmaceutical composition in accordance with claim 4, wherein the concentration of meloxicam or any of the pharmaceutically acceptable salts thereof is 2.0%-15% per dose unit.

7. The pharmaceutical composition in accordance with claim 1, wherein the muscle relaxant is tizanidine and the NSAID is meloxicam, or any of the pharmaceutically acceptable salts thereof.

8. The pharmaceutical composition in accordance with claim 7, wherein the tizanidine dose is 6 mg and the meloxicam dose is 7.5 mg.

9. The pharmaceutical composition in accordance with claim 7, wherein the tizanidine dose is 6 mg and the meloxicam dose is 15 mg.

10. The pharmaceutical composition in accordance with claim 7, wherein the tizanidine dose is 12 mg and the meloxicam dose is 15 mg.

11. The pharmaceutical composition in accordance with claim 1, wherein the inert cores are formed with cellulose or sugars selected from sucrose, lactose, glucose or dextrose.

12. The pharmaceutical composition in accordance with claim 1, wherein the adhesive polymer is selected from hydroxy propyl cellulose, pre-gelatinized starch or hydroxy propyl methyl cellulose.

13. The pharmaceutical composition in accordance with claim 1, wherein the plasticizer is selected from propylene glycol or polyethylene glycol 20000.

14. The pharmaceutical composition in accordance with claim 1, wherein the delaying polymer film is selected from methacrylate derivates.

15. The pharmaceutical composition in accordance with claim 14, wherein the methacrylate derivates are: Eudragit S 100, Eudragit RS, Eudragit RL, Eudragit L30D55, Eudragit 100 55 or Eudragit L 100.

16. The pharmaceutical composition in accordance with claim 1, wherein the surfactant may be an anionic or cationic surfactant.

17. The pharmaceutical composition in accordance with claim 1, wherein the buffer solution may be an acid or basic.

18. The pharmaceutical composition in accordance with claim 17, wherein the solution is selected from: hydrochloric acid, acetic acid, sodium hydroxide or ammonium hydroxide.

19. The use of the pharmaceutical composition in accordance with claim 1 to prepare a drug which may be prescribed for treating spasticity, disorders related to the skeletal muscle and/or muscular ailments, and moderate to severe pain in general, wherein said pharmaceutical composition causes a decreased incidence of gastric damage.

20. A process to obtain the composition of claim 1, **characterized in that** the following is added to the inert cores by spraying:
a) a preparation of the muscle relaxant, at least one adhesive polymer, at least one plasticizer, and at least one surfactant;
b) a second film formed by a delaying polymer film, at least one plasticizer, and at least one buffer solution; and
c) a third film formed by a NSAID, the muscle relaxant, at least one adhesive polymer, at least one plasticizer, and at least one surfactant.

21. The process to elaborate thecoated microspheres in accordance with claim 20, wherein said process is **characterized by** the coating steps being performed continuously and at room temperature.

22. The composition in accordance with claim 1, wherein said composition is **characterized by** being an orally administrated composition.

23. The composition in accordance with claim 1, **characterized in that** said composition is provided in the form of capsules.

24. The composition in accordance with claim 1, **characterized in that** said composition is provided in the form of tablets.

25. The composition in accordance with claim 1, **characterized in that** the synergic effect thereof allows a posology of once or twice a day.

## Patentansprüche

1. Pharmazeutische Zusammensetzung, **gekennzeichnet durch** beschichtete Mikrokügelchen, die:
a) einen inerten Kern, der mit einer ersten Schicht, die aus einem Muskelrelaxans, mindestens einem haftfähigen Polymer und mindestens einem Weichmacher gebildet ist, überzogen ist,
b) eine verzögernde zweite Schicht aus einem Polymer, mindestens einem Weichmacher und einer regulierenden Lösung und
c) eine dritte Schicht, die aus einem NSAID, dem Muskelrelaxans der ersten Schicht, mindestens einem haftfähigen Polymer, mindestens einem Weichmacher und mindestens einem oberflächenaktiven Mittel gebildet ist,
umfassen, wobei das Muskelrelaxans eine modifizierte Freisetzung und das NSAID eine sofortige Freisetzung aufweist.

2. Pharmazeutische Zusammensetzung nach Anspruch 1, wobei das Muskelrelaxans eine Plasmakonzentrationszeit und eine Halbwertszeit aufweist, die sich von der Plasmakonzentrationszeit und der Halbwertszeit des NSAID unterscheiden.

3. Pharmazeutische Zusammensetzung nach Anspruch 1, wobei das Muskelrelaxans Tizanidin oder ein pharmazeutisch verträgliches Salz davon ist.

4. Pharmazeutische Zusammensetzung nach Anspruch 1, wobei das NSAID Meloxicam oder ein pharmazeutisch verträgliches Salz davon ist.

5. Pharmazeutische Zusammensetzung nach Anspruch 3, wobei die Konzentration des Tizanidins oder eines pharmazeutisch verträglichen Salzes davon 0,5 bis 36 % der Dosiseinheit beträgt.

6. Pharmazeutische Zusammensetzung nach Anspruch 4, wobei die Konzentration des Meloxicams oder eines pharmazeutisch verträglichen Salzes davon 2,0 bis 15 % der Dosiseinheit beträgt.

7. Pharmazeutische Zusammensetzung nach Anspruch 1, wobei das Muskelrelaxans Tizanidin und das NSAID Meloxicam oder eines der pharmazeutisch verträglichen Salze von ihnen ist.

8. Pharmazeutische Zusammensetzung nach Anspruch 7, wobei die Tizanidin-Dosis 6 mg und die Meloxicam-Dosis 7,5 mg beträgt.

9. Pharmazeutische Zusammensetzung nach Anspruch 7, wobei die Tizanidin-Dosis 6 mg und die Meloxicam-Dosis 15 mg beträgt.

10. Pharmazeutische Zusammensetzung nach Anspruch 7, wobei die Tizanidin-Dosis 12 mg und die Meloxicam-Dosis 15 mg beträgt.

11. Pharmazeutische Zusammensetzung nach Anspruch 1, wobei die inerten Kerne mit Cellulose oder Zuckern, die aus Sucrose, Lactose, Glucose oder Dextrose ausgewählt sind, gebildet sind.

12. Pharmazeutische Zusammensetzung nach Anspruch 1, wobei das haftfähige Polymer aus Hydroxypropylcellulose, vorverkleisterter Stärke oder Hydroxypropylmethylcellulose ausgewählt ist.

13. Pharmazeutische Zusammensetzung nach Anspruch 1, wobei der Weichmacher aus Propylenglykol oder Polyethylenglykol 20000 ausgewählt ist.

14. Pharmazeutische Zusammensetzung nach Anspruch 1, wobei die verzögernde Polymerschicht aus Methacrylatderivaten ausgewählt ist.

15. Pharmazeutische Zusammensetzung nach Anspruch 14, wobei die Methacrylatderivate Eudragit S 100, Eudragit RS, Eudragit RL, Eudragit L30D55, Eudragit 100 55 oder Eudragit L 100 sind.

16. Pharmazeutische Zusammensetzung nach Anspruch 1, wobei das oberflächenaktive Mittel ein anionisches oder kationisches oberflächenaktives Mittel sein kann.

17. Pharmazeutische Zusammensetzung nach Anspruch 1, wobei die Pufferlösung sauer oder basisch sein kann.

18. Pharmazeutische Zusammensetzung nach Anspruch 17, wobei die Lösung aus Salzsäure, Essigsäure, Natriumhydroxid oder Ammoniumhydroxid ausgewählt ist.

19. Verwendung der pharmazeutischen Zusammensetzung nach Anspruch 1 zur Herstellung eines Arzneimittels, das für die Behandlung von Spastik und Störungen, die mit Skelettmuskulatur- und/oder Muskelschmerzen einhergehen, und allgemein zur Mäßigung schwerer Schmerzen verordnet werden kann, wobei die pharmazeutische Zusammensetzung ein vermindertes Auftreten von Magenschädigungen verursacht.

20. Verfahren zur Herstellung der Zusammensetzung nach Anspruch 1, **dadurch gekennzeichnet, dass** zu den inerten Kernen:
a) eine Zubereitung aus dem Muskelrelaxans, mindestens einem haftfähigen Polymer, mindestens einem Weichmacher und mindestens einem oberflächenaktiven Mittel,
b) eine zweite Schicht, die aus einer verzögernden Polymerschicht, mindestens einem Weichmacher und mindestens einer Pufferlösung gebildet wird, und
c) eine dritte Schicht, die aus einem NSAID, dem Muskelrelaxans, mindestens einem haftfähigen Polymer, mindestens einem Weichmacher und mindestens einem oberflächenaktiven Mittel gebildet wird,
durch Aufsprühen hinzugefügt werden.

21. Verfahren zur Herstellung der beschichteten Mikrokügelchen nach Anspruch 20, **dadurch gekennzeichnet, dass** die Beschichtungsstufen bei Raumtemperatur kontinuierlich durchgeführt werden.

22. Zusammensetzung nach Anspruch 1, **dadurch gekennzeichnet, dass** sie oral zu verabreichen ist.

23. Zusammensetzung nach Anspruch 1, **dadurch gekennzeichnet, dass** sie in Kapselform bereitgestellt wird.

24. Zusammensetzung nach Anspruch 1, **dadurch gekennzeichnet, dass** sie in Tablettenform bereitgestellt wird.

25. Zusammensetzung nach Anspruch 1, **dadurch gekennzeichnet, dass** ihr Synergieeffekt eine Dosierung von einmal oder zweimal täglich erlaubt.

## Revendications

1. Composition pharmaceutique **caractérisée par** des microsphères enrobées comprenant :
a) des coeurs inertes enrobés d'un premier film formé par un myorelaxant, au moins un polymère adhésif, et au moins un plastifiant ;
b) un deuxième film polymère à effet retard, au moins un plastifiant, et une solution de régulation ; et
c) un troisième film formé par un AINS, le myorelaxant du premier film, au moins un polymère adhésif, au moins un plastifiant, et au moins un tensioactif ;
dans laquelle le myorelaxant est à libération modifiée, et l'AINS est à libération immédiate.

2. Composition pharmaceutique selon la revendication 1, dans laquelle le myorelaxant a une concentration plasmatique en fonction du temps et une demi-vie qui sont différentes de la concentration plasmatique en fonction du temps et de la demi-vie de l'AINS.

3. Composition pharmaceutique selon la revendication 1, dans laquelle le myorelaxant est la tizanidine ou l'un quelconque de ses sels pharmaceutiquement acceptables.

4. Composition pharmaceutique selon la revendication 1, dans laquelle l'AINS est le meloxicam ou l'un quelconque de ses sels pharmaceutiquement acceptables.

5. Composition pharmaceutique selon la revendication 3, dans laquelle la concentration de tizanidine, ou de l'un quelconque de ses sels pharmaceutiquement acceptables est de 0,5 % à 36 % par dose unitaire.

6. Composition pharmaceutique selon la revendication 4, dans laquelle la concentration de meloxicam ou de l'un quelconque de ses sels pharmaceutiquement acceptables est de 2,0 % à 15 % par dose unitaire.

7. Composition pharmaceutique selon la revendication 1, dans laquelle le myorelaxant est la tizanidine et l'AINS est le méloxicam, ou l'un quelconque de leurs sels pharmaceutiquement acceptables.

8. Composition pharmaceutique selon la revendication 7, dans laquelle la dose de tizanidine est de 6 mg et la dose de méloxicam est de 7,5 mg.

9. Composition pharmaceutique selon la revendication 7, dans laquelle la dose de tizanidine est de 6 mg et la dose de méloxicam est de 15 mg.

10. Composition pharmaceutique selon la revendication 7, dans laquelle la dose de tizanidine est de 12 mg et la dose de meloxicam est de 15 mg.

11. Composition pharmaceutique selon la revendication 1, dans laquelle les coeurs inertes sont formés avec de la cellulose ou des sucres choisis parmi le saccharose, le lactose, le glucose et le dextrose.

12. Composition pharmaceutique selon la revendication 1, dans laquelle le polymère adhésif est choisi parmi l'hydroxypropylcellulose, l'amidon pré-gélatinisé et l'hydroxypropylméthylcellulose.

13. Composition pharmaceutique selon la revendication 1, dans laquelle le plastifiant est choisi parmi le propylèneglycol et le polyéthylèneglycol 20000.

14. Composition pharmaceutique selon la revendication 1, dans laquelle le film polymère à effet retard est choisi parmi les dérivés de méthacrylate.

15. Composition pharmaceutique selon la revendication 14, dans laquelle les dérivés de méthacrylate sont : l'Eudragit S 100, l'Eudragit RS, l'Eudragit RL, l'Eudragit L30D55, l'Eudragit 100 55 ou l'Eudragit L 100.

16. Composition pharmaceutique selon la revendication 1, dans laquelle le tensioactif peut être un tensioactif anionique ou cationique.

17. Composition pharmaceutique selon la revendication 1, dans laquelle la solution tampon peut être un acide ou une base.

18. Composition pharmaceutique selon la revendication 17, dans laquelle la solution est choisie parmi : l'acide chlorhydrique, l'acide acétique, l'hydroxyde de sodium et l'hydroxyde d'ammonium.

19. Utilisation de la composition pharmaceutique de la revendication 1 pour la préparation d'un médicament qui peut être prescrit pour traiter la spasticité, les troubles liés aux muscles squelettiques et/ou aux affections musculaires, et les douleurs modérées à sévères en général, dans laquelle ladite composition pharmaceutique engendre un impact moins important sur les dommages gastriques.

20. Procédé pour obtenir la composition de la revendication 1, **caractérisé en ce que** ce qui suit est ajouté aux coeurs inertes par pulvérisation :
a) une préparation du myorelaxant, d'au moins un polymère adhésif, d'au moins un plastifiant, et d'au moins un tensioactif ;
b) un deuxième film formé par un film polymère à effet retard, au moins un plastifiant, et au moins une solution tampon ; et
c) un troisième film formé par un AINS, le myorelaxant, au moins un polymère adhésif, au moins un plastifiant, et au moins un tensinactif.

21. Procécé pour élaborer les microsphères enrobées selon la revendication 20, ledit procédé étant **caractérisé en ce que** les étapes d'enrobage sont effectuées en continu et à la température ambiante.

22. Composition selon la revendication 1, ladite composition étant **caractérisée en ce qu'**elle est une composition administrée par voie orale.

23. Composition selon la revendication 1, ladite composition étant **caractérisée en ce qu'**elle est présentée sous la forme de capsules.

24. Composition selon la revendication 1, ladite composition étant **caractérisée en ce qu'**elle est présentée sous la forme de comprimés.

25. Composition selon la revendication 1, **caractérisée en ce que** son effet synergique permet une posologie d'une fois ou deux fois par jour.
